# EUROPÄISCHE PATENTSCHRIFT

(11) **EP 2 659 981 B1**
(45) Veröffentlichungstag und Bekanntmachung des Hinweises auf die Patenterteilung: **23.03.2016**
(21) Anmeldenummer: 13166539.0
(22) Anmeldetag: 03.05.2013
(51) Int. Cl.: B01L 9/06, A61M 5/00, B65B 5/06

(54) **Transport- oder Verpackungsbehälter mit zumindest einer Haltestruktur zum gleichzeitigen Halten einer Mehrzahl von Behältern für medizinische, pharmazeutische oder kosmetische Anwendungen und Verfahren zur Behandlung solcher Behälter**
Transport or packaging container with a support structure for simultaneously holding a plurality of containers for medical, pharmaceutical or cosmetic applications and method for treating such containers
Récipient de transport ou d'emballage avec structure de support pour le support simultané d'une pluralité de récipients pour des applications médicales, pharmaceutiques ou cosmétiques et procédé de traitement de tels récipients

(30) Priorität: 03.05.2012 DE 102012103900; 03.05.2012 DE 102012103898; 29.10.2012 DE 102012110339
(43) Veröffentlichungstag der Anmeldung: 06.11.2013
(73) Patentinhaber: Schott AG, 55122 Mainz (DE)
(72) Erfinder: Wissner, Kai, 69493 Hirschberg (DE); Heiter, Carmen, 9010 St. Gallen (CH); Pawlowski, Edgar, 55271 Stadecken-Elsheim (DE); Fischer, Bastian, 9000 St. Gallen (CH); Dr.-Ing. Lovis, Ralph, 8400 Winterthur (CH); Coulon, Joel, 9000 St. Gallen (CH)
(74) Vertreter: 2K Patentanwälte Blasberg Kewitz & Reichel

(56) Entgegenhaltungen:
- EP-A1- 0 790 063
- US-A- 832 086

## Beschreibung

### Gebiet der Erfindung

Die vorliegende Erfindung betrifft allgemein die gleichzeitige Halterung einer Mehrzahl von Behältern zur Aufbewahrung von Substanzen für medizinische, pharmazeutische oder kosmetische Anwendungen, insbesondere von Fläschchen (Vials) und Ampullen, in einem Transport- oder Verpackungsbehälter und betrifft insbesondere die gleichzeitige Halterung einer Mehrzahl von Behältern in einer Haltestruktur in solcher Weise, dass diese, während diese in einer hierfür vorgesehenen Haltestruktur gehalten werden, in Abfüll- oder Bearbeitungsanlagen weiter verarbeitet werden können, insbesondere in einem Steriltunnel, einer Abfüllanlage für flüssige medizinische oder pharmazeutische Anwendungen oder einem Gefriertrockenschrank zum Lyophilisieren einer wirkstoffhaltigen Flüssigkeit oder dergleichen. Ein weiterer Gesichtspunkt der vorliegenden Erfindung betrifft ferner betrifft ein Verfahren zur Behandlung einer Mehrzahl solcher Behälter mit gleichzeitiger automatisierter Förderung und Übergabe einer Mehrzahl von Behältern an Prozessstationen.

### Hintergrund der Erfindung

Als Behälter zur Aufbewahrung und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die Haltestruktur aus dem Transport- und Verpackungsbehälter, die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten werden. Die Medikamentenbehälter können nicht in dem Transport- oder Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1, US 2011/0277419 A1, WO 2012/025549 A1, WO 2011/015896 A1, WO 2012/007056 A1 und WO 2009/015862 A1 offenbart. Zur Weiterverarbeitung müssen die Medikamentenbehälter jedoch stets vereinzelt werden. Dies sei beispielhaft anhand eines herkömmlichen Verfahrens zur Gefriertrocknung von pharmazeutischen Präparaten in Medikamentenbehältern darstellt, wie dieses beispielsweise in der US 5,964,043 offenbart ist. Zunächst wird die Prozessanlage, ein Steriltunnel, mit den Fläschchen beschickt. Hierzu werden die Fläschchen kopfüber in Transportrahmen eingehängt, die dann durch die Prozessanlage gefördert werden. Zur Vorbehandlung werden die in den Transportrahmen gehaltenen Fläschchen zunächst sterilisiert. Anschließend werden die Transportrahmen mit den darin aufbewahrten Fläschchen gewendet und mit einer Wirkstofflösung befüllt. Anschließend wird auf den oberen Rand der Fläschchen ein Stopfen aufgesetzt, in welchem ein Kanal ausgebildet ist, über den der Flascheninnenraum während der Gefriertrocknung jeweils mit der Kammer des Gefriertrockenschranks kommuniziert.

Zur Gefriertrocknung (auch als Lyophilisation oder Sublimationstrocknung bezeichnet) werden die Fläschchen dann aus dem Transportrahmen entnommen und einzeln in den Gefriertrockenschrank eingebracht. Dabei müssen die Böden der Fläschchen zur Erzielung eines guten Kühleffekts unmittelbar auf einem planar ausgebildeten Kühlboden abgesetzt werden. Wird an dieser Stelle kein vollflächiger unmittelbarer Kontakt gewährleistet, so führt dies zu einer erheblichen Verlängerung des Gefriertrocknungsprozesses, was zu höheren Kosten führt.

Nach der Gefriertrocknung werden die Fläschchen aus dem Gefriertrockenschrank entnommen, die Stopfen heruntergedrückt und ein Metalldeckel auf den Stopfen aufgesetzt und gebördelt oder gecrimpt. Derart verarbeitete Fläschchen werden dann ausgeliefert, beispielsweise indem eine Mehrzahl von Fläschchen gemeinsam von einem Träger aufgenommen und der Träger in einen Transport- und Verpackungsbehälter eingesetzt wird, der zur Auslieferung dann steril verpackt wird.

Der für die Gefriertrocknung notwendige unmittelbare Kontakt zwischen dem Boden der Medikamentenbehälter und dem Kühlboden erfordert herkömmlich eine Behandlung oder Verarbeitung einzelner Behälter, was die Verarbeitungs- und Verpackungskosten erhöht. Eine chargenweise Weiterverarbeitung von Medikamentenbehältern ist herkömmlich nicht möglich. Jedenfalls ist ein unmittelbarer Kontakt der Böden der Medikamentenbehälter, insbesondere der Böden von Fläschchen, bei den herkömmlichen Haltestrukturen nicht möglich.

US 5128105 A, US 2005 / 0013745 A1 und US 2009 / 0238727 A1 offenbaren Haltestrukturen zum gleichzeitigen Halten einer Mehrzahl von Reagenzgläsern. DE 88 05 580 U1 und FR 2595667 offenbaren Haltestrukturen für zerbrechliche Behälter.

US 832086 A offenbart eine Haltestruktur aus einem elastischen Metallband, in dem elastische Halteaufnahmen ausgebildet sind, um darin Behälter zu halten. Das Metallband ist auf der Innenseite eines Kastens befestigt, kann jedoch nicht herausgenommen und wieder eingesetzt werden. US 2598492 offenbart eine vergleichbare Haltestruktur.

US 2011/0132797 A1 offenbart einen Behälter zum Transport von Vials.

EP 0 790 063 A1 offenbart ein vertikal anzuordnendes Gestell zur gleichzeitigen Aufnahme einer Mehrzahl von Spritzenkörpern, die daran gemeinsam behandelt und weiter verarbeitet werden können. Damit die Spritzenkörper vertikal gehalten werden, befindet sich am unteren Ende des Gestells ein Standfuss. Die Spritzenkörper werden in elastische Haltearme eingeclipst. Das Gestell kann jedoch nicht jeweils einzeln von oben her in einen kastenförmigen Transport- oder Verpackungsbehälter eingesteckt werden, was eine umständlichere Prozessführung zur Behandlung oder Verarbeitung der Behälter bedingt.

### Zusammenfassung der Erfindung

Gemäß der Erfindung soll ein Transport- oder Verpackungsbehälter für Behälter zur Aufbewahrung von Substanzen für medizinische, pharmazeutische oder kosmetische Anwendungen dahingehend verbessert werden, dass eine Behandlung oder Weiterverarbeitung der Behälter erleichtert ist, sodass diese insbesondere automatisiert ausgeführt werden kann. Gemäß einem weiteren Gesichtspunkt soll entsprechendes Verfahren zur Behandlung oder Weiterverarbeitung von Behältern bereitgestellt werden, bei dem ein solcher Transport- oder Verpackungsbehälter eingesetzt wird.

Diese Aufgaben werden gemäß der vorliegenden Erfindung durch einen Transport- oder Verpackungsbehälter nach Anspruch 1 sowie durch ein Verfahren nach Anspruch 13 gelöst. Weitere vorteilhafte Ausführungsformen sind Gegenstand der rückbezogenen Unteransprüche.

Somit wird ein Transport- oder Verpackungsbehälter für eine Mehrzahl von Behältern für Substanzen für medizinische, pharmazeutische oder kosmetische Anwendungen, insbesondere von Fläschchen und Ampullen, bereitgestellt, wobei der Transport- oder Verpackungsbehälter rahmen- oder kastenförmig und mit einer umlaufenden Seitenwand ausgebildet ist und wobei in dem Transport- oder Verpackungsbehälter zumindest eine Haltestruktur in Form eines Querstegs zum gleichzeitigen Halten einer Mehrzahl von Behältern in dem Transport- oder Verpackungsbehälter angeordnet ist, wobei an der Vorderseite des Querstegs Paare von elastischen Haltearmen angeordnet sind, welche jeweils eine mittige Ausbauchung aufweisen, um sich jeweils an den Hals eines zu haltenden Behälters anzuschmiegen. Erfingdungsgemäß ist auf der Seitenwand des Transport- oder Verpackungsbehälters oder auf der Seitenwand eines Einsatzes, der in den Transport- oder Verpackungsbehälter eingesetzt ist, eine Mehrzahl von sich vertikal erstreckenden Führungs- und Klemmkanälen ausgebildet ist, wobei Seitenränder des jeweiligen Querstegs in zwei einander gegenüber liegende Führungs- und Klemmkanäle eingesteckt sind, sodass der jeweilige Quersteg jeweils einzeln von oben her in den Transport- oder Verpackungsbehälter einsteckbar ist und in diesem in einer vertikalen Position vorübergehend fixiert ist, um den jeweiligen Quersteg mit den von diesem gehaltenen Behältern in dem Transport- oder Verpackungsbehälter aufzunehmen.

Durch geeignete Dimensionierung und Ausgestaltung der elastischen Haltearme kann die Halterung der Behälter an den Querstegen in einfacher Weise beliebig vorgegeben werden. Insbesondere kann mittels geeigneter Dimensionierung und Ausgestaltung der Haltearme ein geeigneter Reib- oder Formschluss zum Halten der Behälter realisiert werden. Die Halterung der Behälter ist insbesondere so gewählt, dass die Behälter, während diese an den Querstegen gehalten sind, in gewisser Weise bewegt oder verstellt werden können, beispielsweise axial in eine angehobene oder abgesenkte Stellung verschoben und/oder um Ihre Längsachse gedreht werden können. Der Quersteg kann dabei in einer Prozessstation mechanisch fixiert werden und die Behälter können zur Weiterverarbeitung in der Prozessstation geeignet bewegt oder verstellt werden. Anschließend können die Querstege wieder geeignet in einem Transport- oder Verpackungsbehälterabgelegt werden.

Gemäß einer weiteren Ausführungsform sind die Querstege und die Positionen der elastischen Haltearme so auf die Länge der Behälter abgestimmt, dass Böden der Behälter zur Prozessierung frei zugänglich sind, während diese an den Querstegen gehalten sind. Beispielsweise können die Böden unmittelbar an einem Kühlfinger zur Gefriertrocknung des Inhalts der Behälter vollflächig aufliegen. Dabei kann eine gewisse axiale Verschieblichkeit der Behälter, während diese an den Querstegen gehalten sind, von Vorteil sein, um eine vollflächige Anlage zu ermöglichen.

Gemäß einer weiteren Ausführungsform sind die elastischen Haltearme so ausgelegt, dass die Behälter jeweils mit der Unterseite eines verdickten oberen Randabschnitts lose auf den vorderen freien Enden der Haltearme aufliegen. Auf diese Weise können herstellungsbedingte Toleranzen der Abmessungen der Behälter einfach ausgeglichen werden.

Gemäß einem weiteren Gesichtspunkt der vorliegenden Erfindung wird ein entsprechendes Verfahren zur Behandlung oder Verarbeitung von Behältern bereitgestellt, wie in Patentanspruch 13 beansprucht.

Gemäß einer weiteren Ausführungsform des Verfahrens ist eine der Prozessstationen eine Bördeleinrichtung oder eine Crimpstation oder verfügt über eine solche, bei der auf den oberen Rand der Behälter ein Metalldeckel gebördelt oder gecrimpt wird, wobei die Behälter zum Bördeln oder Crimpen in der angehobenen Position von einem Drehteller um ihre Längsachse gedreht werden, während der Metalldeckel gebördelt oder gecrimpt wird, und nach dem Bördeln oder Crimpen wieder in die Öffnungen oder Aufnahmen zurückgeschoben werden. Dabei können die Behälter zum Bördeln oder Crimpen jeweils mittels einer Hubstange in die angehobenen Position angehoben werden, wobei der jeweilige Drehteller auf der Hubstange gelagert ist und wobei der obere Rand der Behälter mit dem darauf aufgesetzten Metalldeckel während des Drehens mittels einer Zentrierscheibe zentriert wird. Während der Behandlung oder Verarbeitung in oder an zumindest einer der Prozessstationen können die Behälter an einem Quersteg, wie vorstehend beschrieben, gehalten werden und in dieser Stellung axial aufwärts in die angehobene Position zur Bearbeitung hochgeschoben werden, in der angehobenen Position bearbeitet werden und abschließend wieder in eine abgesenkte Position zurück geschoben werden, in der die Behälter in den Aufnahmen de Querstegs gehalten sind. Die Behandlung oder Verarbeitung der Behälter kann so flexibler erfolgen, insbesondere während diese auch weiterhin zuverlässig an einem Quersteg gehalten sind. Eine solche Behandlung oder Verarbeitung kann insbesondere ein Reinigungsschritt, ein Sterilisierungsschritt, beispielsweise eine Sterilisierung in einem Steriltunnel, ein Befüllprozess, ein Gefriertrocknungsprozess oder dergleichen sein oder einen solchen Schritt umfassen.

Gemäß einem weiteren Gesichtspunkt soll zumindest an mindestens einer Prozessstation ein Identifikations- oder Nachverfolgungssensor zum Nachweis der Echtheit oder zum Auslesen von Parametern vorhanden sein, der bevorzugt RFID-Chips, RuBee-Chips oder Fluoreszenzmarkierungen auslesen kann.

Gemäß einem weiteren Gesichtspunkt kann die Prozessierung durch optische Sensorik und/oder mechanische Sensorik gesteuert werden. Dazu können besondere Markierungen an den Fläschchen oder Ampullen und/oder an dem Transport- oder Verpackungsbehälter genutzt werden.

Gemäß einer weiteren Ausführungsform wird der Transport- oder Verpackungsbehälter mittels einer Folie oder einer Abdeckung gegen die Umgebung versiegelt oder abgeschlossen.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a-1c: einen Transport- oder Verpackungsbehälter mit einer Mehrzahl von Haltestrukturen gemäß einer ersten Ausführungsform der vorliegenden Erfindung, wobei einzelne Untereinheiten einer Haltestruktur reihenweise in den Transport- oder Verpackungsbehälter eingesteckt und wieder entnommen werden können;
- Fig. 1d: eine Variante zu dem Transport- oder Verpackungsbehälter nach der Fig. 1a, bei dem die Reihen von Haltestrukturen jeweils einzeln in einen herausnehmbaren Einsatz des Transport- oder Verpackungsbehälters eingesteckt sind;
- Fig. 2a: in einem stark vergrößerten Teilschnitt eine Haltestruktur für einen Transport- oder Verpackungsbehälter gemäß einer weiteren Ausführungsform der vorliegenden Erfindung;
- Fig. 2b: in einem stark vergrößerten Teilschnitt die Haltestruktur gemäß der Fig. 2a mit einem darin aufgenommenen Fläschchen;
- Fig. 3a-3c: einen Transport- oder Verpackungsbehälter gemäß einer weiteren Ausführungsform der vorliegenden Erfindung, der an beiden Enden offen ausgebildet und an den offenen Enden mit einer Schutzfolie verschlossen ist; und
- Fig. 4: ein schematisches Flussdiagramm eines Verfahrens zur Behandlung oder Verarbeitung von Behältern gemäß der vorliegenden Erfindung.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

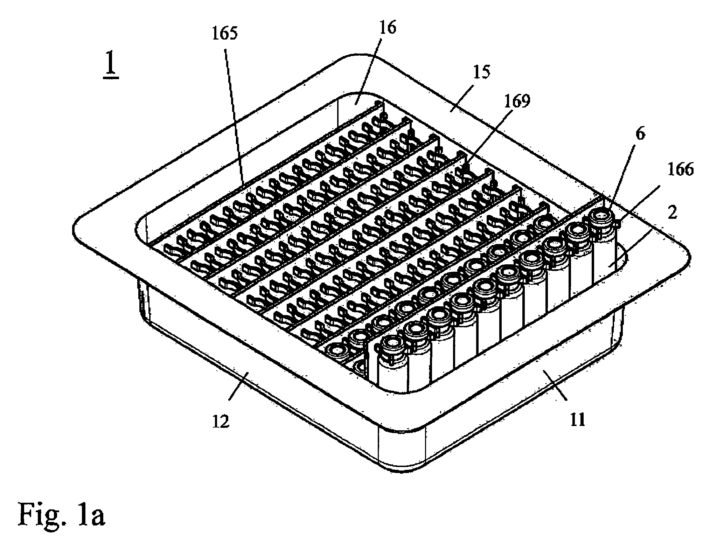

Die Fig. 1a zeigt einen Transport- oder Verpackungsbehälter 1, in den eine Mehrzahl von Untereinheiten in Gestalt von Querstegen 165 eingesteckt sind, an denen jeweils eine Mehrzahl von Fläschchen 2 gehalten sind. Der Transport- oder Verpackungsbehälter dient gemäß der vorliegenden Erfindung, wie nachfolgend beschrieben, der Aufbewahrung einer Mehrzahl von Behältern zur Aufbewahrung von Substanzen für kosmetische, medizinische oder pharmazeutische Anwendungen in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder in regelmäßigen Reihen, die relativ zueinander versetzt angeordnet sind.

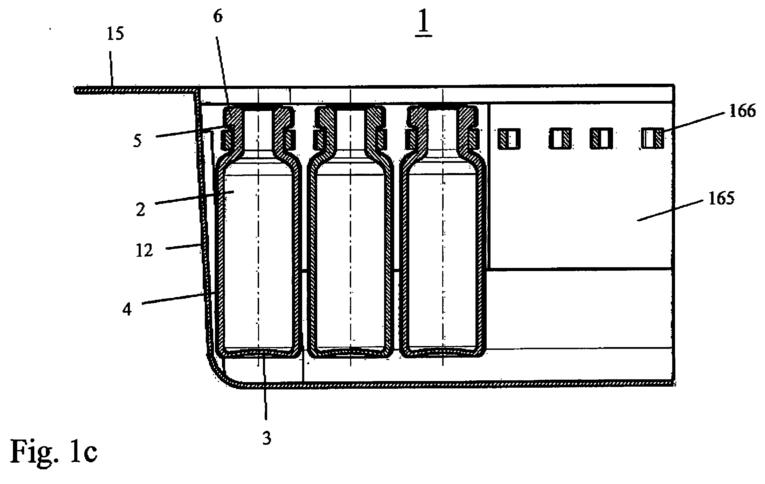

Ein Beispiel für derartige Medikamentenbehälter in Gestalt von Fläschchen (englisch: vial) ist in der Fig. 1c schematisch in einem Längsschnitt dargestellt. Diese weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens 2 gasdicht und geschützt gegen das Eindringen von Verunreinigungen in das Fläschchen mit dem oberen Rand 6 verbunden ist, beispielsweise durch Crimpen oder Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Sollbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummi- oder Kunststoffstopfen verschlossen ist Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Glasbehälter erfindungsgemäß mittels eines Formschlusses oder eines Reibschlusses an einer Halterungsstruktur bzw. Träger fixiert oder von dieser abgestützt. Dieser Form- oder Reibschluss oder diese Abstützung kann im Bereich des verengten Halsabschnitts 5 der Behälter, an dem zylindrischen Seitenwandabschnitt 4 oder im Bereich des unteren Endes der Behälter 2, insbesondere am Boden 3 der Behälter 2 realisiert werden.

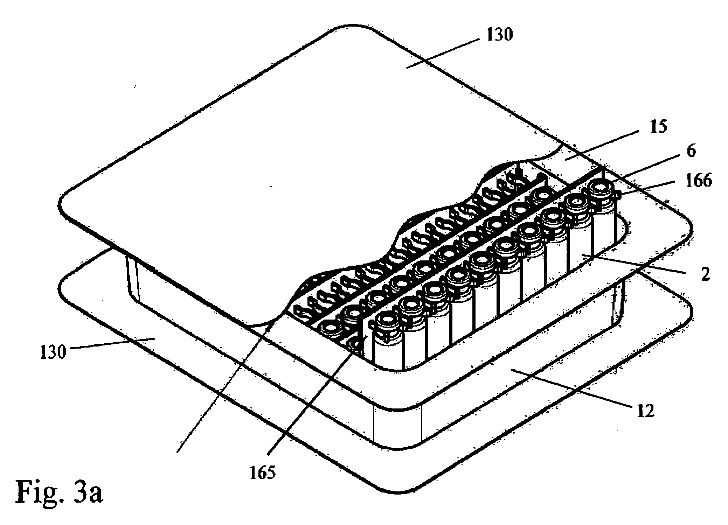

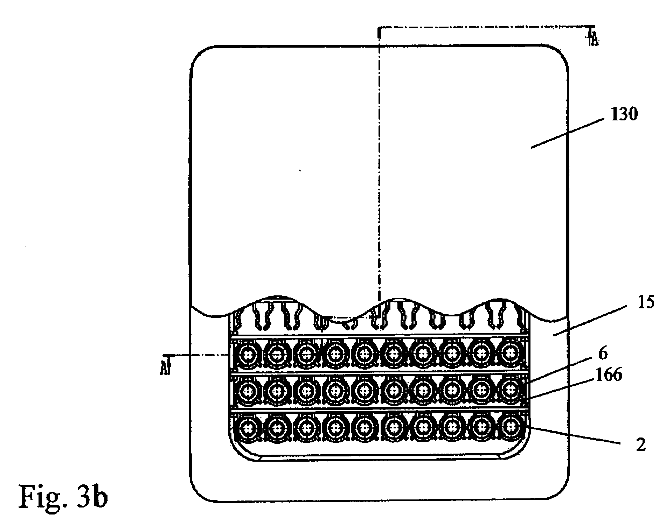

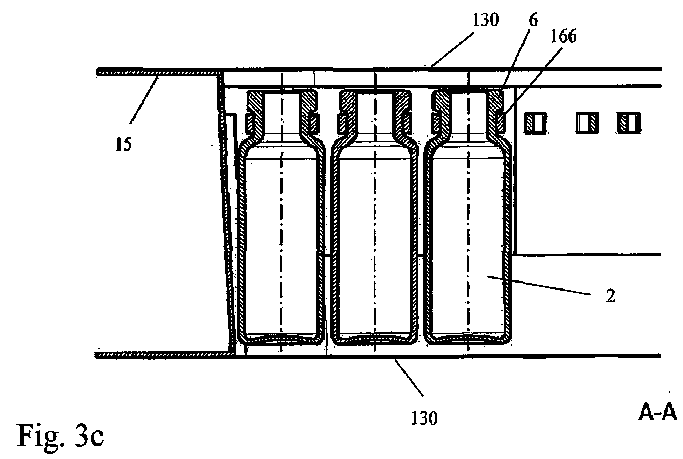

Gemäß der Fig. 1a und 1c weist der Transport- oder Verpackungsbehälter 1 einen geschlossenen, rechteckförmigen Boden 11, eine im Wesentlichen senkrecht von diesem abragende untere Seitenwand 12 und einen davon im Wesentlichen senkrecht abragenden oberen Rand 15 auf. Der Transport- oder Verpackungsbehälter 1 ist nach oben hin offen ausgebildet, sodass die Querstege 165 mit den daran befestigten Fläschchen 2 als Untereinheiten individuell von oben her eingesteckt und wieder herausgenommen werden können. Der Transport- oder Verpackungsbehälter 1 kann mittels einer Folie oder eines Deckels verschlossen werden, wie nachfolgend anhand der Fig. 3a beschrieben. Wenngleich in der Fig. 1a der Boden 11 des Transport- oder Verpackungsbehälters 1 als geschlossen und einstückig mit der Seitenwand 12 ausgebildet dargestellt ist, kann das untere Ende des Transport- oder Verpackungsbehälters 1 auch in der Art des oberen Endes geöffnet ausgebildet sein, insbesondere mit einem flanschartigen unteren Rand in der Art des oberen Rands 15 versehen sein, so dass die Böden der Behälter 2 von der Unterseite des Transport- oder Verpackungsbehälters 1 her frei zugänglich sind, beispielsweise für Verarbeitungsschritte in einem Steriltunnel oder in einem Gefriertrockenschrank.

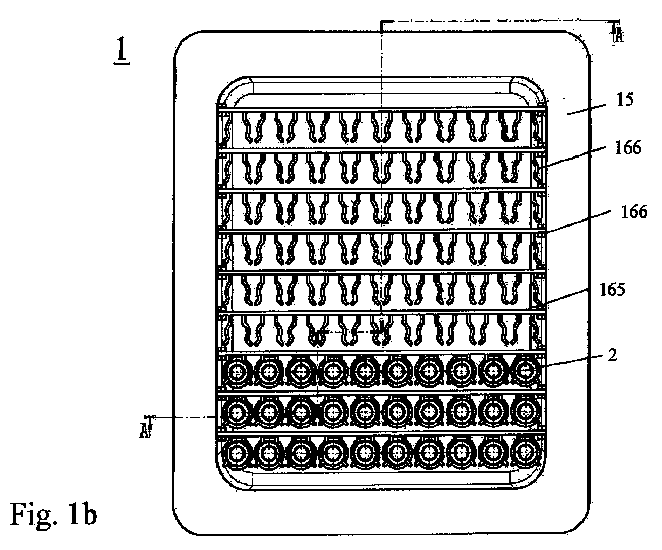

Wie man den Figuren 1a und 1b entnehmen kann, sind auf der Innenseite der unteren Seitenwand 12 in regelmäßigen Abständen zueinander Vorsprünge bzw. Leisten 169 ausgebildet, die senkrecht zum Boden des Transport- oder Verpackungsbehälter 1 verlaufen, wobei zwischen jeweils zwei unmittelbar benachbarten Vorsprüngen 169 ein Kanal ausgebildet ist, in den der Seitenrand des jeweiligen Querstegs 165 eingeführt werden kann. Diese Kanäle sind bevorzugt als Führungs- und Klemmkanäle ausgebildet, in denen die Querstege geklemmt gehalten werden. Dadurch können die Querstege 165 in ihrer horizontalen aber auch vertikalen Position vorübergehend in dem Transport- oder Verpackungsbehälter 1 fixiert werden.

Eine Fixierung der Position der Querstege 165 lässt sich auch durch das formschlüssige Zusammenwirken der Seitenränder der Querstege 165 mit den Führungs- und Klemmkanälen in dem Transport- oder Verpackungsbehälter 1 realisieren. Beispielsweise kann entlang den Seitenrändern zumindest ein Vorsprung oder zumindest eine Vertiefung ausgebildet sein und auf der Innenseite der Vorsprünge 169 korrespondierend dazu eine Vertiefung oder ein Vorsprung ausgebildet sein, die einen Formschluss zur Fixierung der Lage der Querstege 165 an den Seitenwänden 12 des Transport- oder Verpackungsbehälters 1 ermöglichen. Hierzu sind der Transport- oder Verpackungsbehälter 1 und die Querstege 165 bevorzugt aus einem Kunststoff ausgebildet, der ausreichend elastisch ist.

Auf diese Weise können die Querstege 165 in dem Transport- oder Verpackungsbehälter 1 unter gleichmäßigen Abständen zueinander gehalten werden. Die Abstände zwischen den Querstegen 165 sind dabei so bemessen, dass eine möglichst hohe Packungsdichte erzielt werden kann, also der Spalt zwischen den Fläschchenreihen und den benachbarten Querstegen 165 möglichst schmal ist. Dabei verhindern die Querstege 165 eine unmittelbare Berührung von Fläschchen 2 von unmittelbar benachbarten Reihen.

Gemäß den Fig. 1a und 1b sind auf jeweils einer Seite der Querstege 165 in regelmäßigen Abständen zueinander elastische Haltearme 166 angeordnet, die als Haltemittel zum Halten der Fläschchen 2 dienen. Die Haltearme 166 sind bevorzugt einstückig, beispielsweise durch ein Spritzgussverfahren, mit den Querstegen 165 ausgebildet und miteinander fluchtend auf einer Höhe der Querstege 165 angeordnet. Die Abstände zwischen benachbarten Paaren von Haltearmen 166 sind dabei so bemessen, dass eine unmittelbare Kollision von benachbarten Fläschchen unterbunden ist, die Außenoberflächen der Fläschchen 2 also unbeschädigt bleiben.

Die elastischen Haltearme 166 haben jeweils eine mittige Ausbauchung, die sich jeweils an den Hals des zu haltenden Fläschchens 2 anschmiegt. Die Haltearme 166 sind jeweils so auf die Fläschchen 2 abgestimmt und dimensioniert, dass die Fläschchen 2 von den Haltearmen 166 durch einen Reib- oder Formschluss an einer geeigneten Position gehalten werden. Dabei können die Fläschchen 2, während diese an den Querstegen 165 gehalten sind, weiterhin bearbeitet und gehandhabt werden, da der größte Teil der Fläschchen 2 im gehaltenen Zustand auch weiterhin zugänglich ist. Wie man der Fig. 1a entnehmen kann, sind die Fläschchen 2 im gehaltenen Zustand von oben her zugänglich, ist deren Boden von der Unterseite der Querstege 165 her zugänglich (was für eine Kühlung mittels eines anliegenden Kühlfingers wichtig ist) und ist deren gesamte Vorderseite ungehindert zugänglich, wenn die Querstege 165 aus dem Transport- oder Verpackungsbehälter 1 entnommen sind. Die Haltearme 166 können ferner so ausgelegt sein, dass die Fläschchen 2, während diese von den Haltearmen 166 gehalten sind, gedreht und/oder axial verschoben werden können. Hierzu wird durch geeignete Materialwahl der elastischen Haltearme 166 für eine geeignete Reibpaarung mit dem Material der Fläschchen 2 gesorgt, sodass die Fläschchen 2 einerseits zuverlässig von den Haltearmen 166 gehalten sind, also nicht aufgrund ihres Eigengewichts, verrutschen, andererseits mit nicht allzu hohem Kraftaufwand verstellt werden können, während diese von den Haltearmen 166 gehalten sind. Diese freie Drehbarkeit der Fläschchen 2 kann beispielsweise zum Bördeln eines auf den oberen Rand der Fläschchen 2 aufgesetzten Metalldeckels verwendet werden, wie nachfolgend ausführlicher anhand der Fig. 5 beschrieben.

Die Halterung der Fläschchen 2 an einem Quersteg 165 ist beispielhaft in der Fig. 1c dargestellt. Die Fläschchen 2 sind im Bereich des verengten Halsabschnittes 5 und unterhalb des verbreiterten oberen Rands 6 (Rollrand) gehalten, sodass die Fläschchen 2 von den Haltearmen 166 axial gesichert gehalten sind. Die Böden 3 der Fläschchen 2 stehen über den unteren Rand der Querstege 165 vor, was jedoch nicht zwingend ist. Gemäß weiteren Ausführungsformen kann der obere Rand 6 der Fläschchen über den oberen Rand der Querstege 165 vorstehen.

Im Sinne der vorliegenden Erfindung stellen die Querstege 165 Untereinheiten dar, die jeweils mit den daran gehaltenen Fläschchen 2 separat gehandhabt oder prozessiert werden können, jedoch gleichzeitig eigenständige Haltestrukturen darstellen, um mehrere Fläschchen 2 geeignet zu halten. Die Querstege 165 können beispielsweise einzeln gegriffen (z.B. mit Greifer) und weiterverarbeitet bzw. behandelt werden. Dabei sind die Böden der Fläschchen 2 vollständig von unten her zugänglich sind, wenn die Querstege 165 aus dem Transport- oder Verpackungsbehälter 1 herausgenommen sind.

Bei der Ausführungsform nach den Figuren 1a und 1b sind die als Untereinheiten wirkenden Querstege 165 einzeln in den Transport- oder Verpackungsbehälter 1 eingesteckt und über diesen miteinander verbunden. Die einzelnen Querstege 165 können jedoch auch gemeinsam in einen Halterahmen oder Haltekasten eingesteckt und von diesem gehalten sein, der dann als Einsatz in den Transport- oder Verpackungsbehälter 1 eingesetzt und wieder aus diesem entnommen werden kann.

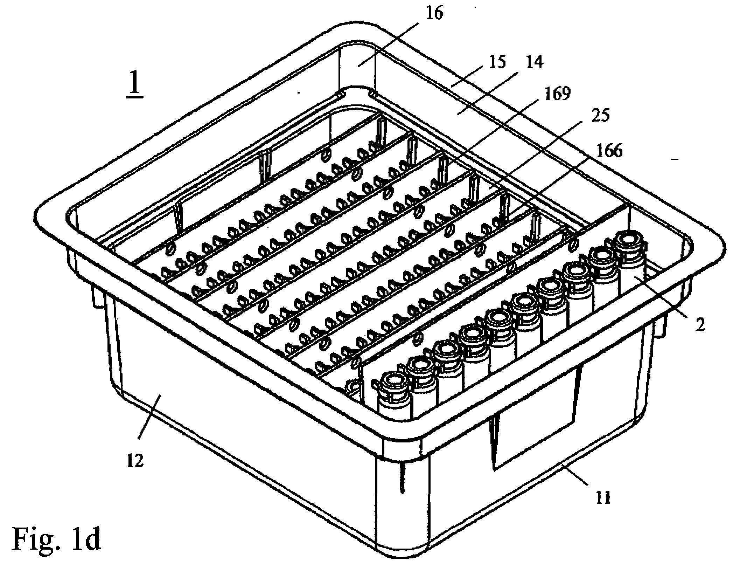

Dies ist beispielhaft in der Fig. 1d dargestellt, bei der die Vorsprünge 169 zum Fixieren der Querstege nicht unmittelbar auf den unteren Seitenwänden 12 des Transportbehälter- und Verpackungsbehälters 1 ausgebildet sind sondern vielmehr auf den Seitenwänden eines rahmenartigen oder kastenartigen Einsatzes 25, der in den Transport- oder Verpackungsbehälter 1 eingesetzt werden kann. Dabei können die Querstege 165 in ähnlicher Weise wie vorstehend beschrieben in den rahmenartigen oder kastenartigen Einsatz 25 eingesteckt und wieder aus diesem entnommen werden, unabhängig davon, ob dieser sich in dem Transport- oder Verpackungsbehälter 1 oder außerhalb davon befindet. Die Unterseite des rahmenartigen oder kastenartigen Einsatzes 25 kann dabei geschlossen oder geöffnet ausgebildet sein.

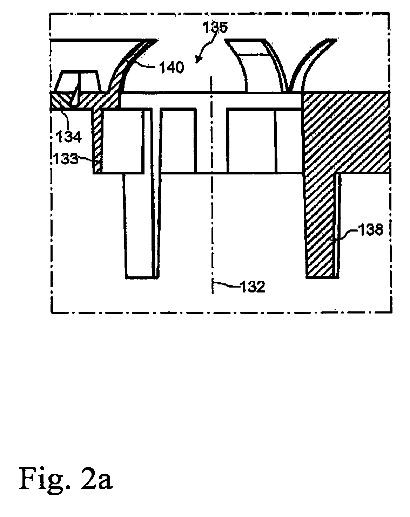

Die Fig. 2a zeigt in einem stark vergrößerten Teilschnitt eine weitere Variante für Haltemittel zum Halten der Fläschchen (nicht dargestellt), die bei sämtlichen Ausführungsformen nach der vorliegenden Erfindung eingesetzt werden können. Hierzu sind an einem Quersteg, wie vorstehend beschrieben (nicht dargestellt), statt der elastischen Haltearme 166 elastische Haltezungen 140 vorgesehen, die vertikal von einem an dem Quersteg vorgesehenen horizontalen Träger 134 abragen und sich ausgehend von der Oberseite des horizontaln Trägers 134 bogenförmig einwärts in die Öffnung 135 erstrecken. Die Haltezungen 140 sind bevorzugt aus einem ausreichend flexiblen oder elastischen Kunststoff ausgebildet. Alternativ können die Haltezungen 140 auch relativ steif ausgebildet sein, jedoch so beweglich an der Oberseite des Trägers 134 gelagert sein, dass diese beim Einführen der Behälter elastisch aus der Öffnung 135 weggeschwenkt oder zurück geklappt werden.

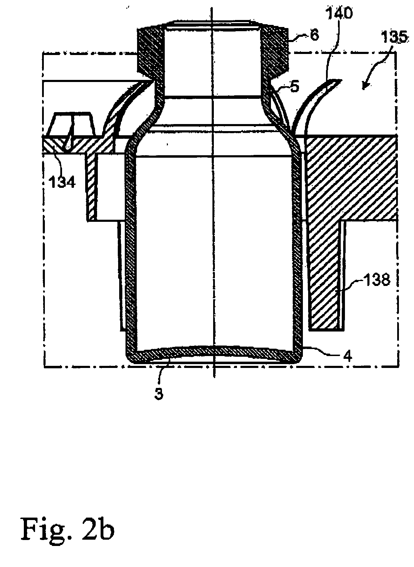

Die Halterung der Fläschchen wird bei dieser Ausführungsform im Übergangsbereich des verengten Halsabschnitts 5 zum verbreiterten oberen Rand 6 realisiert, wie in dem stark vergrößerten Teilschnitt gemäß der Fig. 2b beispielhaft dargestellt. Insbesondere liegt die Unterseite des Rands 6 der Behälter im Übergangsbereich zum verengten Halsabschnitt 5 lose auf den oberen Enden von Haltezungen 140 auf, wobei die Fläschchen gleichwohl axial gesichert gehalten sind. Die Fläschchen können gemäß einer ersten Ausführungsform von unten her in die Öffnungen 135 des Trägers 134 eingeführt werden können. Beim Einführen der Fläschchen in die Öffnungen 135 kommt es zu einem elastischen Verbiegen der elastischen Haltezungen 140. Gemäß einer weiteren Ausführungsform können die Fläschchen grundsätzlich auch von oben her in die Öffnungen 135 des Trägers 134 eingerührt werden, um an dem Quersteg gehalten zu werden.

Wie man in der Fig. 2b erkennen kann, besteht zwischen den Haltezungen 140 (vgl. linker Bildteil) und dem verengten Halsabschnitt 5 ein Luftspalt, der ein radiales Spiel ermöglicht. Aufgrund dieser Halterung der Fläschchen mit radialem Spiel besteht dabei, je nach konkreter Ausbildung des Fläschchens, noch die Möglichkeit, die von den Haltezungen 140 gehaltenen Fläschchen axial zu verschieben, d.h. in Längsrichtung der Fläschchen, beispielsweise solange, bis die Böden 3 von allen von dem Quersteg gehaltenen Fläschchen unter dem gleichen Abstand zum Quersteg gehalten werden, um gemeinsam eine Ebene aufzuspannen.

Gemäß der Fig. 2b ist das Fläschchen soweit in die Öffnung 135 eingeschoben, dass der verbreiterte Rand 6 auf den vorderen Enden der Halterungen genau an dem Übergangsbereich zwischen dem verengten Halsabschnitt 5 und dem verbreiterten oberen Rand 6 abgestützt ist. Dies lässt sich beispielsweise durch Einschieben der Fläschchen von unten her in die Öffnungen 135 des Trägers 134 und anschließendes Herabdrücken der Fläschchen bewerkstelligen, und zwar solange, bis die vorderen Enden der Haltezungen genau an dem Übergangsbereich zwischen dem verengten Halsabschnitt 5 und dem verbreiterten oberen Rand 6 anliegen. In der Haltestellung gemäß der Fig. 2b ist jedenfalls bei der großen Mehrzahl der fixierten Fläschchen ein gewisser radialer Abstand zwischen dem stufenartigen Übergangsbereich zwischen dem oberen Rand 6 und dem verengten Halsabschnitt 5 und vorderen Ende der Haltezungen 140 vorgesehen. Auf diese Weise können in einem gewissen Umfang Fertigungstoleranzen der Fläschchen in axialer Richtung und auch Fertigungstoleranzen in radialer Richtung kompensiert werden und somit auch Fläschchen mit unterschiedlichen Durchmessern im Bereich des verengten Halsabschnitts 5 von ein- und demselben Quersteg gehalten werden.

Die Oberseite oder die Ober- und Unterseite eines Einsatzes 25, wie vorstehend beschrieben, oder auch eines Transport- oder Verpackungsbehälters 1, wie beispielhaft anhand der Fig. 1a beschrieben, kann bzw. können von einer sterilen, gasdurchlässigen Schutzfolie überzogen sein, die aufgeklebt und bei Bedarf abziehbar ist. Dies ist beispielhaft in der Fig. 3a für eine Verpackungseinheit dargestellt, die von einem beidseitig offenen Transportbehälter und einer darin aufgenommen Haltestruktur gemäß der Fig. 1a ausgebildet ist und auf der Ober- und Unterseite mittels einer auf den flanschartigen Rand 15 aufgeklebten Schutz- oder Verpackungsfolie 130 verschlossen ist. Bei der Schutzfolie 130 kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Fläschchen 2 durch die Folie 130 hindurch ermöglicht.

Die Fläschchen 2 können in einen solchen Transport- oder Verpackungsbehälter 1 auch nur vorübergehend und ohne Füllung versiegelt aufbewahrt werden. Über die gasdurchlässige Schutzfolie 130 kann ein Gas zur Sterilisierung der noch offenen Fläschchen in das Innere des Transport- oder Verpackungsbehälters 1 eindringen. Ein solcher Transport- oder Verpackungsbehälter 1 kann dann später in einer Prozessstation oder einem Steriltunnel wieder geöffnet werden, um die Fläschchen weiter zu handhaben oder zu prozessieren.

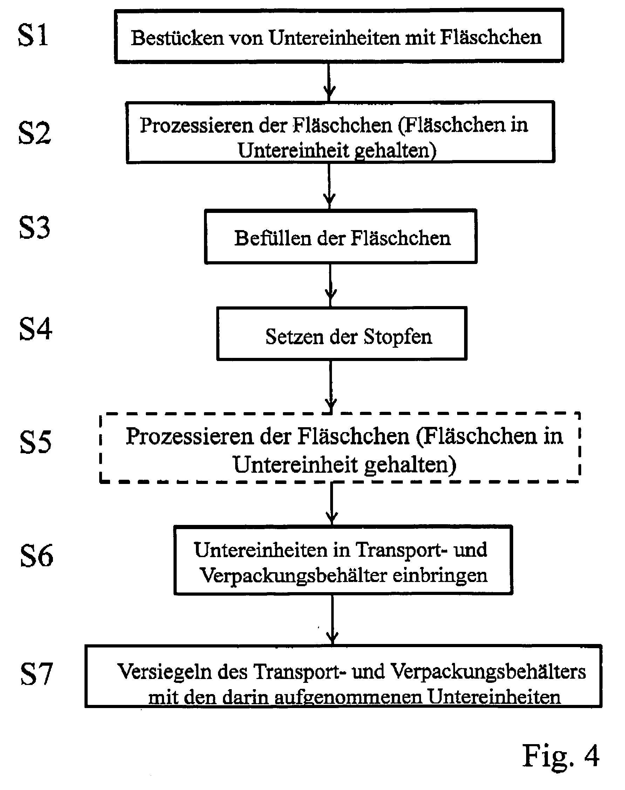

Nachfolgend wird anhand' der Fig. 4 schematisch ein Verfahren zur Behandlung oder Verarbeitung von Behältern, die zur Aufbewahrung von Substanzen für medizinische, pharmazeutische oder kosmetische Anwendungen dienen oder diese enthalten, beschrieben. Zunächst werden die als Untereinheiten dienenden Querstege in dem Schritt S1 mit Fläschchen bestückt. Diese können grundsätzlich bereits befüllt sein, in welchem Fall die Fläschchen in den nachfolgenden Prozessschritten beispielsweise verschlossen oder deren Inhalt zunächst gefriergetrocknet werden. Der Fig. 4 ist jedoch der Fall zugrunde gelegt, dass die Fläschchen, während diese an den Untereinheiten gehalten sind, befüllt werden sollen.

In dem Prozessschritt S2 werden die Fläschchen weiterbehandelt, beispielsweise gewaschen und anschließend getrocknet und/oder sterilisiert, während diese an den Untereinheiten gehalten sind. Dieser Prozessschritt ist optional.

Anschließend werden die Fläschchen in dem Prozessschritt S3 befüllt, sei es mit einer einen Wirkstoff enthaltenden Flüssigkeit oder einem Pulver. Anschließend werden die Fläschchen in dem Prozessschritt S4 verschlossen, beispielsweise mit einem Stopfen oder einem Metalldeckel, der auch gecrimpt oder gebördelt werden kann. Diese Prozessschritte S3 und S4 erfolgen, während die Fläschchen an den als Untereinheiten dienenden Querstegen gehalten sind.

In dem optionalen Prozessschritt S5, der auch vor dem Schritt S4 ausgeführt werden kann, können die Fläschchen weiter verarbeitet werden, während diese an den Querstegen gehalten sind. Bei einem solchen Prozessschritt kann es sich beispielsweise um eine Gefriertrocknung handeln, während der die Querstege an einen Gefriertrockenschrank übergeben werden, in welchem die Gefriertrocknung erfolgt. Zwecksmäßig sind dabei die Querstege so ausgelegt, dass sämtliche Böden der Fläschchen, während diese an den Querstegen gehalten sind, von der Unterseite her frei zugänglich sind, sodass diese unmittelbar auf einem Kühlfinger des Gefriertrockenschranks vollflächig aufliegen können. Selbstverständlich kann es sich bei einem solchen Prozessschritt auch um beliebige andere Verarbeitungsschritte handeln, beispielsweise eine thermische Behandlung oder Bestrahlung. Dabei ist von Vorteil, dass bei den Querstegen im Sinne der vorliegenden Erfindung und wie vorstehend beschrieben, die Behälter größtenteils frei zugänglich sind, insbesondere von deren Ober- und Unterseite sowie von der Vorderseite der Untereinheiten her, wie vorstehend beispielsweise anhand der Figuren 1a bis 3c beschrieben.

In dem anschließenden Prozessschritt S6 werden die Querstegen wieder in einen Transport- oder Verpackungsbehälter eingebracht, wie beispielsweise in den Figuren 1a oder 1d dargestellt.

Somit können die Fläschchen erfindungsgemäß chargenweise behandelt oder verarbeitet werden. Einer die Prozesse aufwändig machenden Entnahme aus einem Träger und einer Vereinzelung bedarf es grundsätzlich nicht. Hierzu sind die Querstege erfindungsgemäß so ausgelegt, dass die Fläschchen an diesem form- oder reibschlüssig gehalten werden können. Insbesondere werden die Fläschchen in hierfür geeignet ausgebildeten Aufnahmen der Querstege gehalten. Die Querstege können so ausgelegt sein, dass die Fläschchen, während diese an diesen gehalten werden, verschoben oder gedreht oder in vergleichbarer Weise verstellt oder bewegt werden können. Dies lässt sich durch entsprechende Auslegung der Halterung der Fläschchen, beispielsweise des Form- oder Reibschlusses, in einfacher Weise gewährleisten. Somit können die Fläschchen beispielsweise zum Bördeln oder Crimpen eines Metalldeckels, der auf deren oberen Rand aufgesetzt ist, gedreht werden, während diese an einem Quersteg gehalten sind.

Die Fläschchen können zur Behandlung oder Verarbeitung an oder in einer Prozessstation in der jeweiligen Aufnahme eines Querstegs in Längsrichtung in eine angehobene Position verschoben werden, in welcher die weitere Behandlung oder Verarbeitung dann erleichtert ist.

Beispielsweise kann in dieser angehobenen Position der Boden der Fläschchen vollständig zugänglich sein oder der obere Rand der Fläschchen über den oberen Rand eines Querstegs oder eines Transport- oder Verpackungsbehälters geeignet weit vorstehen, sodass erst in der angehobenen Position eine Behandlung oder Verarbeitung möglich ist. Zweckmäßig werden die Fläschchen in dieser angehobenen Position im Bereich ihrer zylindrischen Seitenwand oder aber eines verengten Halsabschnitts unterhalb des oberen Rands oder an ihrem oberen Rand gehalten, was von der jeweiligen Prozessstation abhängen kann.

Die Fläschchen können in der angehobenen Position weiterhin in den Aufnahmen eines Querstegs aufgenommen sein, jedoch auf einer zusätzlichen Abstützfläche abgestützt oder mittels einer zusätzlichen Halte- oder Greifeinrichtung gehalten werden, während diese an oder in der Prozessstation behandelt oder verarbeitet werden. Die Haltemittel an einem Quersteg sind hierzu so ausgelegt, dass diese in der angehobenen Position die Fläschchen nicht halten, jedenfalls nicht mit einer dem Gewicht der Fläschchen entsprechenden ausreichenden Haltekraft. Jedoch brauchen auch bei einer solchen Ausführungsform die Fläschchen nicht vollständig aus einem Quersteg entnommen zu werden, sodass diese weiterhin chargenweise behandelt oder verarbeitet werden können, jedoch dennoch rascher an einen nachfolgenden Prozessschritt übergeben werden können.

## Patentansprüche

1. Transport- oder Verpackungsbehälter (1) für eine Mehrzahl von Behältern (2) für Substanzen für medizinische, pharmazeutische oder kosmetische Anwendungen, wobei der Transport- oder Verpackungsbehälter (1) rahmen- oder kastenförmig und mit einer umlaufenden Seitenwand (12) ausgebildet ist und in dem Transport- oder Verpackungsbehälter zumindest eine Haltestruktur in Form eines Querstegs (165) zum gleichzeitigen Halten einer Mehrzahl von Behältern (2) in dem Transport- oder Verpackungsbehälter (1)angeordnet ist, wobei an der Vorderseite des Querstegs (165) Paare von elastischen Haltearmen (166) angeordnet sind, welche jeweils eine mittige Ausbauchung aufweisen, um sich jeweils an den Hals (5) eines zu haltenden Behälters (2) anzuschmiegen, **dadurch gekennzeichnet, dass** auf der Seitenwand (12) des Transport- oder Verpackungsbehälters (1) oder auf der Seitenwand eines Einsatzes (25), der in den Transport- oder Verpackungsbehälter (1) eingesetzt ist, eine Mehrzahl von sich vertikal erstreckenden Führungs- und Klemmkanälen ausgebildet ist, wobei Seitenränder des jeweiligen Querstegs (165) in zwei einander gegenüber liegende Führungs- und Klemmkanäle eingesteckt sind, sodass der jeweilige Quersteg (165) jeweils einzeln von oben her in den Transport- oder Verpackungsbehälter (1) einsteckbar ist und in diesem in einer vertikalen Position vorübergehend fixiert ist, um den jeweiligen Quersteg (165) mit den von diesem gehaltenen Behältern (2) in dem Transport- oder Verpackungsbehälter (1) aufzunehmen.

2. Transport- oder Verpackungsbehälter nach Anspruch 1, wobei der Transport- oder Verpackungsbehälter (1) einen geschlossenen, rechteckförmigen Boden (11) aufweist und die Seitenwand (12) von diesem abragt, wobei
die Führungs- und Klemmkanäle auf der Innenseite der Seitenwand (12) von Paaren von parallel zueinander und in regelmäßigen Abständen zueinander verlaufenden Vorsprüngen oder Leisten (169) ausgebildet sind, um den jeweiligen Quersteg zu fixieren.

3. Transport- oder Verpackungsbehälter nach Anspruch 1 oder 2, wobei der Transport- oder Verpackungsbehälter mit einer sterilen, gasdurchlässigen Schutzfolie verschlossen ist.

4. Transport- oder Verpackungsbehälter (1) nach einem der vorhergehenden Ansprüche, wobei die elastischen Haltearme (166) jeweils so auf die zu haltenden Behälter (2) abgestimmt sind, dass diese durch einen Reib- oder Formschluss an dem jerweiligen Quersteg (2) gehalten sind.

5. Transport- oder Verpackungsbehälter (1) nach einem der vorhergehenden Ansprüche, wobei die elastischen Haltearme (166) so ausgelegt sein, dass die Behälter (2), während diese von den Haltearmen (166) gehalten sind, gedreht und/oder axial verschoben werden können.

6. Transport- oder Verpackungsbehälter (1) nach einem der vorhergehenden Ansprüche, wobei der jeweiligen Quersteg (165) und die Positionen der elastischen Haltearme (166) so auf die Länge der zu haltenden Behälter (2) abgestimmt sind, dass Böden der zu haltenden Behälter zur Prozessierung frei zugänglich sind, während diese an dem jeweiligen Quersteg gehalten sind.

7. Transport- oder Verpackungsbehälter (1) nach einem der vorhergehenden Ansprüche, wobei die elastischen Haltearme (166) so ausgelegt sind, dass die Behälter (2) im Bereich des Halses (5) und unterhalb eines sich dem Hals anschließenden verbreiterten oberen Rands (6) gehalten sind, sodass die Behälter (2) von den elastischen Haltearmen (166) axial gesichert gehalten sind.

8. Transport- oder Verpackungsbehälter (1) nach einem der Ansprüche 1 bis 6, wobei die elastischen Haltearme (166) so ausgelegt sind, dass die Behälter (2) jeweils mit der Unterseite eines verdickten oberen Randabschnitts (6) lose auf den vorderen freien Enden der Haltearme (166) aufliegen.

9. Transport- oder Verpackungsbehälter (1) nach einem der vorhergehenden Ansprüche, wobei Abstände zwischen benachbarten Paaren von elastischen Haltearmen (166) des jeweiligen Querstegs so bemessen sind, dass eine unmittelbare Kollision von benachbarten Behältern (2) unterbunden ist.

10. Transport- oder Verpackungsbehälter (1) nach einem der vorhergehenden Ansprüche, wobei die elastischen Haltearme (166) an der Vorderseite des jeweiligen Quersteges (165) unter regelmäßigen Abständen zueinander angeordnet sind.

11. Transport- oder Verpackungsbehälter (1) nach einem der vorhergehenden Ansprüche, wobei die elastischen Haltearme (166) einstückig mit dem jeweiligen Quersteg (165) aus einem Kunststoff ausgebildet sind.

12. Transport- oder Verpackungsbehälter (1) nach einem der vorhergehenden Ansprüche, wobei die elastischen Haltearme (166) miteinander fluchtend auf einer Höhe des jeweiligen Querstegs (165) angeordnet sind.

13. Verfahren zur Behandlung oder Verarbeitung von Behältern (2), die zur Aufbewahrung von Substanzen für medizinische, pharmazeutische oder kosmetische Anwendungen dienen oder diese enthalten, unter Verwendung eines Transport- oder Verpackungsbehälters nach einem der vorhergehenden Ansprüche, in welchem zumindest ein Quersteg (165) aufgenommen ist, an dem eine Mehrzahl von Behältern (2) gehalten sind, bei welchem Verfahren:
der jeweilige Quersteg (165) aus dem Transport- oder Verpackungsbehälter entnommen wird, während die Mehrzahl von Behältern (2) an dem Quersteg gehalten werden;
die Behälter (2), während diese von dem jeweiligen Quersteg gehalten werden, behandelt oder verarbeitet werden; und
der jeweiligen Quersteg mit den daran gehaltenen Behältern (2) nach der Behandlung oder Verarbeitung wieder von oben her in den Transport- oder Verpackungsbehälter (1) eingesteckt wird, sodass die Seitenränder des jeweiligen Querstegs in zwei einander gegenüber liegende Führungs- und Klemmkanäle auf der Seitenwand (12) des Transport- oder Verpackungsbehälters (1) oder des Einsatzes (25) eingesteckt werden und der jeweilige Quersteg (165) in dem Transport- oder Verpackungsbehälter (1) in vertikaler Position vorübergehend fixiert wird.

14. Verfahren nach Anspruch 13, wobei die Behälter (2) zur Behandlung oder Verarbeitung an oder in einer Prozessstation in der jeweiligen Aufnahme des jeweiligen Querstegs (165) in Längsrichtung in eine angehobene Position verschoben werden, in welcher die weitere Behandlung oder Verarbeitung erfolgt, wobei der Transport- oder Verpackungsbehälter (1) nach der Behandlung oder Verarbeitung der Behälter (2) mittels einer Folie (130) oder einer Abdeckung gegen die Umgebung versiegelt oder abgeschlossen wird.

## Claims

1. A transport or packaging container (1) for a plurality of containers (2) for substances for medical, pharmaceutical or cosmetic applications, wherein the transport or packaging container (1) is frame-shaped or box-shaped and formed with a circumferential side wall (12) and wherein at least one supporting structure in the form of a transverse web (165) is disposed in the transport or packaging container (1) for concurrently holding a plurality of containers (2) in the transport or packaging container (1), wherein pairs of resilient holding arms (166) are disposed on the front side of the transverse web (165), each comprising a central bulge in order to respectively snuggle to the neck (5) of a container (2) to be supported,
**characterized in that** on the side wall (12) of the transport or packaging container (1) or on the side wall of an insert (25) inserted into the transport or packaging container (1) a plurality of guide and clamping channels are formed extending vertically, wherein side edges of the respective transverse web (165) are inserted into two guide and clamping channels disposed opposite to each other so that the respective transverse web (165) can be inserted individually and from above into the transport container (1) and can be retained therein temporarily in a vertical position for accommodating the respective transverse web (165) and the containers (2) held by it in the transport or packaging container (1).

2. The transport or packaging container of claim 1, wherein the transport or packaging container (1) comprises a closed, rectangular bottom (11) and the side wall (12) protrudes therefrom, wherein
the guide and clamping channels on the inner side of the side wall (12) are formed by pairs of protrusions or ridges (169) extending in parallel with each other and at regular intervals, for retaining the respective transverse web.

3. The transport or packaging container of claim 1 or 2, wherein the transport or packaging container is sealed by a sterile, gas-permeable protective foil.

4. The transport or packaging container (1) of any of the preceding claims, wherein each of the resilient holding arms (166) is matched to the container (2) to be supported such that it is held by friction or in a positive-fit manner on the respective transverse web (2).

5. The transport or packaging container (1) of any of the preceding claims, wherein the resilient holding arms (166) are configured such that the containers (2) can be rotated and/or displaced axially while being held by the holding arms (166).

6. The transport or packaging container (1) of any of the preceding claims, wherein the respective transverse web (165) and the positions of the resilient holding arms (166) are matched to the length of the containers (2) to be supported such that bottoms of the containers to be held are freely accessible for a processing, while they are held on the respective transverse web.

7. The transport or packaging container (1) of any of the preceding claims, wherein the resilient holding arms (166) are configured such that the containers (2) are held in the region of the neck (5) and below an expanded upper rim (6) that follows the neck so that the containers (2) are held by the resilient holding arms (166) so as to be secured in axial direction.

8. The transport or packaging container (1) of any of claims 1 to 6, wherein the resilient holding arms (166) are configured such that the containers (2) respectively rest loosely with the lower side of an expanded upper rim portion (6) on the front free ends of the holding arms (166).

9. The transport or packaging container (1) of any of the preceding claims, wherein distances between adjacent pairs of resilient holding arms (166) of the respective transverse web are dimensioned such that a direct collision of adjacent containers (2) is prevented.

10. The transport or packaging container (1) of any of the preceding claims, wherein the resilient holding arms (166) are disposed on the front side of the respective transverse web (165) spaced apart from each other at regular intervals.

11. The transport or packaging container (1) of any of the preceding claims, wherein the resilient holding arms (166) are formed of a plastics material and integrally with the respective transverse web (165).

12. The transport or packaging container (1) of any of the preceding claims, wherein the resilient holding arms (166) are disposed aligned with one another at a given level of the respective transverse web (165).

13. A process for the treatment or processing of containers (2), which serve for the storage of substances for medical, pharmaceutical or cosmetic applications or contain such substances, using a transport or packaging container according to any of the preceding claims, in which at least transverse web (165) is accommodated on which a plurality of containers (2) are held, in which process:
the respective transverse web (165) is removed from the transport or packaging container while the plurality of containers (2) are held on the transverse web;
the containers (2) are treated or processed while being supported by the respective transverse web; and
the respective transverse web together with the containers (2) supported by it are inserted again from above into the transport or packaging container (1) after the treatment or processing so that the side edges of the respective transverse web are inserted into two guide and clamping channels disposed opposite to each other on the side wall (12) of the transport or packaging container (1) or of the insert (25) and that the respective transverse web (165) is retained temporarily in a vertical position in the transport or packaging container (1).

14. The process of claim 13, wherein the containers (2) are displaced in a longitudinal direction into a raised position to be treated or processed in the respective receptacle of the respective transverse web (165) at or in a processing station where a further treatment or processing of the containers is performed in the raised position, wherein the transport or packing container (1) is sealed or closed from the surroundings by means of a foil (130) or cover after the treatment or processing of the containers (2).

## Revendications

1. Un récipient de transport ou d'emballage (1) pour une pluralité de récipients (2) pour des applications médicales, pharmaceutiques ou cosmétiques, dans lequel le récipient de transport ou d'emballage (1) présente la forme d'un cadre ou d'une boîte et est formée avec une paroi latérale circonférentielle (12) et dans lequel au moins une structure de soutien sous la forme d'une paroi transversale (165) est disposée dans le récipient de transport ou d'emballage (1) pour maintenir conjointement une pluralité de récipients (2) au sein du récipient de transport ou d'emballage (1), dans lequel des paires de bras de support élastique (166) sont disposés sur le côté avant des paires de parois transversales (165), chacune comprenant un bombement central pour se nicher au niveau du nez (5) d'un récipient (2) devant être soutenu,
**caractérisé en ce qu**'une pluralité de canaux de guidage et d'attache sont formés et s'étendent verticalement sur la paroi latérale (12) du récipient de transport ou d'emballage (1) ou sur la paroi latérale d'un insert (25) insérée au sein du récipient de transport ou d'emballage (1), dans lequel des côtés latéraux de la paroi transversale respective (165) sont insérés à l'intérieur de deux canaux de guidage et d'attache disposés en face l'un à l'autre de telle manière que la paroi transversale respective (165) peut être insérée individuellement et par le dessus à l'intérieur du récipient de transport (1) et peut y être maintenue provisoirement dans une position verticale pour recevoir la paroi transversale respective (165) ainsi que les récipients (2) maintenue par celle-ci au sein du récipient de transport ou d'emballage (1).

2. Le récipient de transport ou d'emballage de la revendication 1, dans lequel le récipient de transport ou d'emballage (1) comporte un fond fermé, rectangulaire (11) et la paroi latérale (12) la surplombant, dans lequel
les canaux de guidage et d'attache disposé sur le côté intérieur de la paroi latérale (12) sont formées en paires de protubérances ou saillies (169) s'étendant parallèlement les uns avec les autres et à intervalles régulier, pour le maintien de la barre transversale respective.

3. Le récipient de transport ou d'emballage de la revendication 1 ou 2, dans lequel le récipient de transport ou d'emballage est scellé par une feuille protectrice stérile perméable à l'air.

4. Le récipient de transport ou d'emballage (1) de l'une quelconque des revendications précédentes, dans lequel chaque bras de support élastique (166) est apparié au récipient (2) devant être maintenu de façon à ce qu'il puisse être maintenu par friction ou d'une manière adéquate sur la barre transversale respective (2).

5. Le récipient de transport ou d'emballage (1) de l'une quelconque des revendications précédentes, dans lequel les bras de support élastique (166) sont configurés de telle manière que les récipients (2) peuvent tourner et/ou être déplacés axialement tout en étant retenus par les bras de support (166).

6. Le récipient de transport ou d'emballage (1) de l'une quelconque des revendications précédentes, dans lequel la paroi transversale respective (165) et les positions des bras de support élastique (166) sont appariés à la longueur des récipients (2) devant être maintenus de telle manière que les fonds des récipients devant être maintenus sont librement accessible pour traitement, tout en étant maintenus sur leur paroi transversale respective.

7. Le récipient de transport ou d'emballage (1) de l'une quelconque des revendications précédentes, dans lequel les bras de support élastique (166) sont configurés de telle façon que les récipients (2) sont maintenus dans la région du col (5) et en dessous un côté supérieur étendu (6) suivant le col de telle manière que les récipients (2) sont maintenus par les bras de support élastiques (166) de façon à être fixés suivant une direction axiale.

8. Le récipient de transport ou d'emballage (1) de l'une quelconque des revendications 1 à 6, dans lequel les bras de support élastique (166) sont configurés de telle façon que les récipients (2) reposent respectivement sans serrage avec le côté inférieur d'une partie de côté supérieur étendu (6) sur les extrémités libres avant des bras de support (166).

9. Le récipient de transport ou d'emballage (1) de l'une quelconque des revendications précédentes, dans lequel on dimensionne les distances entres des paires adjacentes de bras de support élastique (166) de la barre transversale respective pour éviter une collision directe entre deux récipients adjacent (2).

10. Le récipient de transport ou d'emballage (1) de l'une quelconque des revendications précédentes, dans lequel les bras de support élastique (166) sont disposés sur le côté avant de la paroi transversale respective (165), espacées l'une à l'autre d'un intervalle régulier.

11. Le récipient de transport ou d'emballage (1) de l'une quelconque des revendications précédentes, dans lequel les bras de support élastique (166) sont formés en matière plastique et de fabrication avec la paroi transversale respective (165).

12. Le récipient de transport ou d'emballage (1) de l'une quelconque des revendications précédentes, dans lequel les bras de support élastique (166) sont disposés en alignement les uns aux autres à un niveau donné de la paroi transversale respective (165).

13. Un procédé pour le traitement ou la transformation de récipients (2), qui servent pour le stockage de substances cosmétiques, pharmaceutiques, ou médicales, en utilisant un récipient de transport ou d'emballage selon l'une quelconque des revendications précédentes, dans lequel se trouve la paroi transversale (165) sur laquelle sont maintenues une pluralité de récipients (2), dans lequel :
la paroi transversale respective (165) est enlevée du récipient de transport ou d'emballage alors que la pluralité de récipients (2) sont maintenus sur la paroi transversale ;
les récipients (2) sont traités ou font l'objet d'un traitement tandis qu'ils sont maintenus par leur paroi transversale respective ; et
la paroi transversale respective est insérée, avec les récipients (2) qu'elle supporte, par le dessus dans le récipient de transport ou d'emballage (1) à la suite du traitement de telle manière que les côtés latéraux de la paroi transversale respective sont insérés dans deux canaux de guidage et d'attache disposés l'un face à l'autre sur la paroi latérale (12) du récipient de transport ou d'emballage (1) ou de l'insert (25) et en ce que la paroi transversale respective (165) est retenue provisoirement en position verticale dans le récipient de transport ou d'emballage (1).

14. Le procédé de la revendication 13, dans lequel deux récipients (2) sont déplacés suivant une direction longitudinale vers une position élevée pour être traités dans le réceptacle respective de la paroi transversale respective (165) ou au sein d'une station de traitement où un traitement supplémentaire des récipients est effectué dans la position élevée, dans lequel le récipient de transport ou d'emballage (1) est scellé ou fermé vis à vis de son environnement au moyen d'une feuille (130) ou d'une couverture à la suite du traitement des récipients.
